# EUROPEAN PATENT SPECIFICATION

(11) **EP 4 169 519 B1**
(45) Date of publication and mention of the grant of the patent: **12.11.2025**
(21) Application number: 21827129.4
(22) Date of filing: 15.06.2021
(51) Int. Cl.: A61K 9/50, A61K 35/39, A61K 47/34, A61K 47/36, A61P 3/10, A61K 35/12, C12N 5/071

(54) **ENCAPSULATED PANCREATIC ISLET**
EINGEKAPSELTE PANKREAS-INSEL
ÎLOT PANCREATIQUE ENCAPSULÉ

(30) Priority: 17.06.2020 JP 2020104236
(43) Date of publication of application: 26.04.2023
(73) Proprietor: Otsuka Pharmaceutical Factory, Inc., Naruto-shi, Tokushima 772-8601 (JP)
(72) Inventor: AZUMA, Koji, Naruto-shi, Tokushima 772-8601 (JP); IIZUKA, Naho, Naruto-shi, Tokushima 772-8601 (JP); TAMURA, Hirofumi, Naruto-shi, Tokushima 772-8601 (JP)
(74) Representative: Hoffmann Eitle
(86) International application number: PCT/JP2021/022611
(87) International publication number: WO 2021/256451

(56) References cited:
- WO-A1-02/32437
- WO-A1-2013/023013
- JP-A- 2008 515 434
- JP-A- 2013 501 528
- JP-A- 2014 506 926
- LEUNG A ET AL: "Tissue transplantation by stealth@?Coherent alginate microcapsules for immunoisolation", BIOCHEMICAL ENGINEERING JOURNAL, ELSEVIER, AMSTERDAM, NL, vol. 48, no. 3, 15 February 2010 (2010-02-15), pages 337 - 347, XP026886641, ISSN: 1369-703X, [retrieved on 20091020], DOI: 10.1016/J.BEJ.2009.10.007

## Description

### Technical Field

Technologies relating to an encapsulated pancreatic islet are disclosed.

### Background Art

Pancreatic islet transplantation is a means of treatment for diabetes with a relatively low burden on the human body; however, strong immunosuppression is required for long-term survival of transplanted pancreatic islets, and immunosuppressants have been administered to transplant patients in medical practice. However, since the administration of immunosuppressants has the risk of side effects, a method for transplanting a large number of pancreatic islets into an abdominal cavity by isolating pancreatic islets from immune cells by microcapsulation has been discussed. This method does not require immunosuppression, or can reduce the administration amount; however, sufficient therapeutic results have not yet been obtained.

### Citation List

### Patent Literature

PTL 1: WO2001/052871

### Non-patent Literature

| | |
|---|---|
| NPL 1: | Methods in Enzymology, Vol. 137, 575-580, 1988 |
| NPL 2: | Transplantation, Vol. 53, 1180-1183, No. 6, June 1992 |
| NPL 3: | Bioartificial Pancreas, Vol. 98, No. 6, 1996, 1417-1422 |
| NPL 4: | Drug Delivery System, Vol. 12, No. 2, 1997 |
| NPL 5: | Journal of Biomedical Materials Research Part B: Applied Biomaterials, 2013, Vol. 101B, Issue 2, 258-268 |

WO 02/32437 A1 relates to a method of preparing a xenotransplantable porcine islet preparation capable upon xenotransplantation of producing porcine insulin in an appropriate recipient mammal.
Leung A. et al., Biochem. Eng. J. 48(3), 2010, 337-347 address the prevention of immune attacks on transplanted tissue by means of encapsulation and specifically describe pancreatic islet cells enclosed in a three-layer alginate/polycation/alginate membrane.

### Summary of Invention

### Technical Problem

An object is to provide an improved encapsulated pancreatic islet.

### Solution to Problem

As a means for achieving the above object, the following invention is provided.

A formulation comprising a core covered with a five-layered membrane,
wherein
the core comprises one or more pancreatic islets, and
the first, third, and fifth membranes from the inner side of the five-layered membrane contain alginic acid, and the second and fourth membranes from the inner side contain polyornithine.

### Advantageous Effects of Invention

The present invention provides a means effective for the treatment of diabetes.

### Brief Description of Drawings

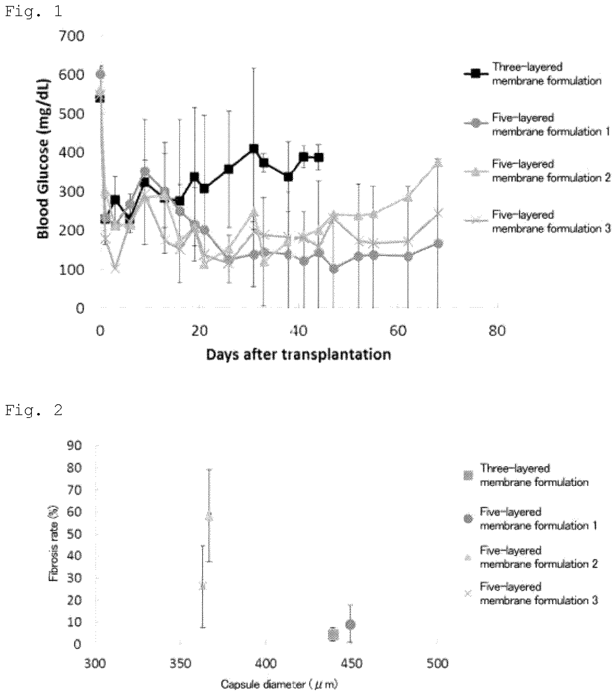

Fig. 1 shows the measurement results of blood glucose levels in diabetic mouse models after the administration of encapsulated pancreatic islets. ■ indicates the results of the administration of a three-layered membrane formulation, ● indicates the results of the administration of five-layered membrane formulation 1, ▲ indicates the results of the administration of five-layered membrane formulation 2, and X indicates the results of the administration of five-layered membrane formulation 3.
Fig. 2 shows the measurement results of fibrosis rate after the administration of encapsulated pancreatic islets. ■ indicates the results of the administration of a three-layered membrane formulation, ● indicates the results of the administration of five-layered membrane formulation 1, A indicates the results of the administration of five-layered membrane formulation 2, and X indicates the results of the administration of five-layered membrane formulation 3. Description of Embodiments

It is claimed that the formulation comprise a core covered with a five-layered membrane, wherein the core comprises one or more pancreatic islets; and the first, third, and fifth membranes from the inner side of the five-layered membrane contain alginic acid, and the second and fourth membranes from the inner side contain polyornithine. Such a formulation enables the blood glucose level to be maintained in an appropriate range for a longer period of time. In one embodiment, the formulation may be covered with more than five membranes (e.g., six, seven, eight, nine, or ten membranes). The formulation covered with a five-layered membrane means a formulation that is prepared by a method comprising the step of forming a membrane described below five times.

Any type of a pancreatic islet can be used, without particular limitation. The pancreatic islet preferably contains insulin-producing β-cells, glucagon-containing α-cells, somatostatin-secreting delta cells, and pancreatic polypeptide-containing cells (PP cells). Most of the pancreatic islet is preferably formed of insulin-producing β-cells. The source of pancreatic islet can be selected according to the purpose thereof; the pancreatic islet is preferably derived from a human, a pig, a mouse, a rat, a monkey, or a dog. In one embodiment, the pancreatic islet is preferably derived from a pig, and a pancreatic islet derived from a neonatal pig (e.g., 3-day-old to 4-week-old, or 7-day-old to 3-week-old) is preferred. A pancreatic islet can be obtained by employing any method known in this technical field. For example, a method using Liberase as a digestive enzyme (T.J. Cavanagh et al., Transplantation Proceedings, 30, 367 (1998)) can be preferably used.

The size of the pancreatic islet is preferably 50 µm or more to 400 µm or less, and more preferably 50 µm or more to 350 µm or less. The size of the pancreatic islet can be measured using a microscopic micrometer. The pancreatic islet preferably contain β-cells at a concentration of 10% or more. The upper limit of the ratio of the β-cells is not particularly limited; it is, for example, 80%.

The membrane containing alginic acid means that the membrane is mainly composed of alginic acid. The membrane containing polyornithine means that the membrane is mainly composed of polyornithine.

The formulation preferably comprises 1 to 5 pancreatic islets in a capsule.

The kind of alginic acid constituting the membrane of alginic acid is not particularly limited. The kind of polyornithine constituting the membrane of polyornithine is not particularly limited.

The formulation preferably has an average diameter of 400 µm or more, and more preferably 420 µm or more, from the viewpoint of inhibiting fibrosis. The upper limit of the average diameter is not particularly limited; for example, it can be set to 700 µm or less, 600 µm or less, or 500 µm or less. The average diameter can be measured using a measurement method employed in the Examples described below.

The above formulation is useful for the treatment of diabetes. The kind of diabetes is not limited, and examples include type 1 diabetes. In addition, the above formulation is effective for the treatment of patients with difficulty in blood glucose control, regardless of the cause.

Pancreatic islets can be produced by any method. In one embodiment, the pancreatic islets can be preferably produced by a method comprising the following steps (a) to (g):
(a) preparing a sodium alginate solution A containing pancreatic islets;
(b) adding the sodium alginate solution A to a divalent cation solution dropwise, and collecting gelled particles;
(c) adding the particles collected in step (b) to a poly-L-ornithine solution A, followed by stirring for a predetermined period of time and then collecting particles;
(d) adding the particles collected in step (c) to a sodium alginate solution B, followed by stirring for a predetermined period of time and then collecting particles;
(e) adding the particles collected in step (d) to a poly-L-ornithine solution B, followed by stirring for a predetermined period of time and then collecting particles;
(f) adding the particles collected in step (e) to a sodium alginate solution C, followed by stirring for a predetermined period of time and then collecting particles; and
(g) adding the particles collected in step (f) to a sodium citrate solution, followed by stirring for a predetermined period of time and then collecting particles.

The sodium alginate concentration of the sodium alginate solution A is not particularly limited. For example, it is preferably 1 w/v% or more to 3 w/v% or less.

It is preferable that the sodium alginate solution A comprise pancreatic islets at a concentration of 10,000 IEQ/mL or more, from the viewpoint of improving the glucose responsiveness of pancreatic islets after encapsulation. IEQ means the number of standard pancreatic islets having a diameter of 150 µm. The concentration of pancreatic islets is preferably 11,000 IEQ/mL or more, or 12,000 IEQ/mL or more. The upper limit of the concentration of pancreatic islets is not particularly limited. For example, it is 30,000 IEQ/mL or less, or 25,000 IEQ/mL or less.

The specific method for preparing the sodium alginate solution A is not limited as long as the sodium alginate solution A is prepared so that pancreatic islets are contained at a concentration mentioned above. For example, the sodium alginate solution A can be obtained by dissolving an appropriate amount of sodium alginate in a physiological saline solution, adding an appropriate amount of pancreatic islets thereto, and optionally performing stirring.

The divalent cation solution used in step (b) is not limited, as long as gelled particles (capsules) encapsulating pancreatic islets can be obtained by adding the sodium alginate solution A dropwise to the divalent cation solution. Examples of divalent cations constituting the divalent cation solution include salts that release divalent metal ions in an aqueous solution (e.g., calcium chloride, calcium lactate, barium chloride, and strontium chloride). In one embodiment, a preferred salt is calcium chloride, and a preferred divalent cation solution is a calcium chloride solution.

The concentration of divalent cations in the divalent cation solution is not particularly limited. For example, it can be set in the range of 50 to 500 mM.

The style of dropwise addition of the sodium alginate solution A to the divalent cation solution is not limited as long as gelled particles encapsulating pancreatic islets can be obtained. In one embodiment, the sodium alginate solution A is preferably added dropwise to the divalent cation solution through an appropriately sized needle.

The gelled particles can be collected by any method. For example, the gelled particles can be collected by allowing the divalent cation solution in which the gelled particles are formed to stand for a certain period of time, and removing the supernatant; or by repeating such a step.

The poly-L-ornithine solution A used in step (c) is not particularly limited as long as it is capable of coating the gelled particles formed in step (b). For example, the poly-L-ornithine solution A can be prepared by dissolving poly-L-ornithine in a physiological saline solution.

The concentration of poly-L-ornithine in the poly-L-ornithine solution A is not particularly limited; it is preferably 0.05 w/v% or more to less than 1 w/v% or less, from the viewpoint of adjusting the average diameter of the formulations (capsules) to 50 µm or more to 400 µm or less. In one embodiment, the concentration of poly-L-ornithine in the poly-L-ornithine solution A is preferably 0.9 w/v% or less or 0.8 w/v% or less.

Step (c) of "adding the particles collected in step (b) to a poly-L-ornithine solution A" also includes an embodiment in which the poly-L-ornithine solution A is added to the gelled particles collected in step (b). The stirring speed and time in step (c) is not limited. For example, stirring is preferably performed for 1 to 20 minutes.

The gelled particles can be collected in any manner in step (c). For example, the gelled particles can be collected by allowing the poly-L-ornithine solution A to stand for a certain period of time, and removing the supernatant. The collected gelled particles preferably have a structure in which the surface of a gelled layer due to alginic acid is coated with poly-L-ornithine.

The sodium alginate solution B used in step (d) may have a concentration that is the same or different from that of the sodium alginate solution A used in step (a). In one embodiment, the sodium alginate concentration of the sodium alginate solution B is preferably lower than the sodium alginate concentration of the sodium alginate solution A in terms of ease of coating operation. For example, when the sodium alginate concentration of the sodium alginate solution A is 1 w/v% or more to 3 w/v% or less, the sodium alginate concentration of the sodium alginate solution B is preferably 0.1 w/v% or more to 0.3 w/v% or less.

Step (d) of "adding the particles collected in step (c) to a sodium alginate solution B" also includes an embodiment in which the sodium alginate solution B is added to the gelled particles collected in step (c). The stirring speed and time in step (d) is not limited. For example, stirring is preferably performed for 1 to 15 minutes.

The gelled particles can be collected in any manner in step (d). For example, the gelled particles can be collected by allowing the sodium alginate solution B to stand for a certain period of time, and removing the supernatant. The collected gelled particles preferably have a structure in which the surface of a gelled layer due to alginic acid is coated with poly-L-ornithine, and a gelled layer due to alginic acid is further formed thereon.

In one embodiment, it is preferable that the particles collected in step (c) be added to a poly L-ornithine solution A', stirred for a predetermined period of time, and then collected before being added to the sodium alginate solution B; and then further added to the sodium alginate solution B in step (d). The poly-L-ornithine solution A' may have a concentration that is the same as or different from that of the poly-L-ornithine solution A. In one embodiment, the poly-L-ornithine concentration of the poly-L-ornithine solution B is preferably lower than the poly-L-ornithine concentration of the poly-L-ornithine solution A. For example, the poly-L-ornithine concentration of the poly-L-ornithine solution A' is preferably 0.01 w/v% or more to less than 0.05 w/v%.

Steps (e) and (f) are basically a repetition of steps (c) and (d). The concentration of poly-L-ornithine in the poly-L-ornithine solution B is not particularly limited; it is preferably 0.01 w/v% or more to less than 0.05 w/v%.

Step (e) of "adding the particles collected in step (d) to a poly-L-ornithine solution B" also includes an embodiment in which the poly-L-ornithine solution B is added to the gelled particles collected in step (d). The stirring speed and time in step (e) is not limited. For example, stirring is preferably performed for 1 to 20 minutes.

The gelled particles can be collected in any manner in step (e). For example, the gelled particles can be collected by allowing the poly-L-ornithine solution A to stand for a certain period of time, and removing the supernatant. The collected gelled particles preferably have a structure in which the surface of a gelled layer due to alginic acid is coated with poly-L-ornithine, alginic acid, and poly-L-ornithine.

The sodium alginate solution C used in step (f) may have a concentration that is the same as or different from that of the sodium alginate solution B used in step (d). For example, the sodium alginate concentration of the sodium alginate solution C is preferably 0.1 w/v% or more to 0.3 w/v% or less.

Step (f) of "adding the particles collected in step (e) to a sodium alginate solution C" also includes an embodiment in which the sodium alginate solution C is added to the gelled particles collected in step (e). The stirring speed and time in step (f) is not limited. For example, stirring is preferably performed for 1 to 15 minutes.

The gelled particles can be collected in any manner in step (f). For example, the gelled particles can be collected by allowing the sodium alginate solution C to stand for a certain period of time, and removing the supernatant. The collected gelled particles preferably have a structure in which the surface of a gelled layer due to alginic acid is coated with poly-L-ornithine, alginic acid, poly-L-ornithine, and alginic acid.

The sodium citrate solution used in step (g) preferably contains sodium citrate at a concentration of 0.5 to 3 w/v%. The sodium citrate solution can be added for the purpose of stimulating insulin release by chelating cations in the gelled particles to put the gel close to a liquid state.

Step (g) of "adding the particles collected in step (d) to a sodium citrate solution" also includes an embodiment in which the sodium citrate solution is added to the gelled particles collected in step (d). The stirring speed and time in step (e) is not limited. For example, stirring is preferably performed for 1 to 15 minutes.

The gelled particles can be collected in any manner in step (e). For example, the gelled particles can be collected by allowing the sodium citrate solution to stand for a certain period of time, and removing the supernatant. The collected gelled particles are considered to contain an inner content that is more of a liquid state than the outer content.

### Examples

The present invention is described in more detail by means of Examples below; however, the present invention is not limited to these Examples.

### 1. Preparation of three-layered membrane formulation (not claimed)

Neonatal pig-derived pancreatic islets isolated from a 14-day-old pig were suspended in a 1.7 w/v% sodium alginate solution (PRONOVA) at a concentration of 16,000 IEQ/mL. The resultant was passed through a needle, and added dropwise to a 109 mM calcium chloride solution while cutting the suspension by airflow. Solidified alginate beads were collected from the calcium chloride solution, and added to a 0.075 w/v% poly-L-ornithine (PLO) (molecular weight 5,000 to 15,000) solution, followed by stirring for 10 minutes. The beads were collected, and further added to a 0.038 w/v% PLO solution, followed by stirring for 6 minutes and collecting. The beads were then added to a 0.17 w/v% sodium alginate solution, followed by stirring for 6 minutes. Finally, the beads were added to a 1.6% sodium citrate solution, followed by stirring for 2 minutes, thus obtaining encapsulated pancreatic islets (formulation) covered with a three-layered membrane: an alginic acid membrane, a polyornithine membrane, and an alginic acid membrane. The resulting encapsulated pancreatic islets were cultured in a CO₂ incubator at 37°C for 25 days.

### 2. Preparation of five-layered membrane formulation 1

Neonatal pig-derived pancreatic islets isolated from a 14-day-old pig were suspended in a 1.7 w/v% sodium alginate solution (PRONOVA) at a concentration of 16,000 IEQ/mL. The resultant was passed through a needle, and added dropwise to a 109 mM calcium chloride solution while cutting the suspension by airflow. Solidified alginate beads were collected from the calcium chloride solution, and added to a 0.075 w/v% poly-L-ornithine (PLO) (molecular weight 5,000 to 15,000) solution, followed by stirring for 10 minutes. The beads were collected, and further added to a 0.038 w/v% PLO solution, followed by stirring for 6 minutes and collecting. The beads were then added to a 0.17 w/v% sodium alginate solution, followed by stirring for 6 minutes. The beads were collected, and added again to a 0.038 w/v% PLO solution, followed by stirring for 6 minutes and collecting. The beads were then added to a 0.17 w/v% sodium alginate solution, followed by stirring for 6 minutes. Finally, the beads were added to a 1.6% sodium citrate solution, followed by stirring for 2 minutes, thus obtaining encapsulated pancreatic islets (five-layered membrane formulation 1) covered with a five-layered membrane: an alginic acid membrane, a polyornithine membrane, an alginic acid membrane, a polyornithine membrane, and an alginic acid membrane. The resulting encapsulated pancreatic islets were cultured in a CO₂ incubator at 37°C for 25 days.

### 3. Preparation of five-layered membrane formulation 2

Neonatal pig-derived pancreatic islets isolated from a 14-day-old pig were suspended in a 1.7 w/v% sodium alginate solution (PRONOVA) at a concentration of 16,000 IEQ/mL. The resultant was passed through a needle, and added dropwise to a 109 mM calcium chloride solution while cutting the suspension by airflow. Solidified alginate beads were collected from the calcium chloride solution, and added to a 0.10 w/v% poly-L-ornithine (PLO) (molecular weight 5,000 to 15,000) solution, followed by stirring for 10 minutes. The beads were collected, and further added to a 0.05 w/v% PLO solution, followed by stirring for 6 minutes and collecting. The beads were then added to a 0.17 w/v% sodium alginate solution, followed by stirring for 6 minutes. The beads were collected, and added again to a 0.038 w/v% PLO solution, followed by stirring for 6 minutes and collecting. The beads were then added to a 0.17 w/v% sodium alginate solution, followed by stirring for 6 minutes.

Finally, the beads were added to a 1.6% sodium citrate solution, followed by stirring for 2 minutes, thus obtaining encapsulated pancreatic islets (five-layered membrane formulation 2) covered with a five-layered membrane: an alginic acid membrane, a polyornithine membrane, an alginic acid membrane, polyornithine membrane, and an alginic acid membrane. The resulting encapsulated pancreatic islets were cultured in a CO₂ incubator at 37°C for 25 days.

### 4. Preparation of five-layered membrane formulation 3

Neonatal pig-derived pancreatic islets isolated from a 14-day-old pig were suspended in a 1.7 w/v% sodium alginate solution (PRONOVA) at a concentration of 16,000 IEQ/mL. The resultant was passed through a needle, and added dropwise to a 109 mM calcium chloride solution while cutting the suspension by airflow. Solidified alginate beads were collected from the calcium chloride solution, and added to a 0.15 w/v% poly-L-ornithine (PLO) (molecular weight 5,000 to 15,000) solution, followed by stirring for 10 minutes. The beads were collected, and further added to a 0.075 w/v% PLO solution, followed by stirring for 6 minutes and collecting. The beads were then added to a 0.17 w/v% sodium alginate solution, followed by stirring for 6 minutes. The beads were collected, and added again to a 0.038 w/v% PLO solution, followed by stirring for 6 minutes and collecting. The beads were then added to a 0.17 w/v% sodium alginate solution, followed by stirring for 6 minutes. Finally, the beads were added to a 1.6% sodium citrate solution, followed by stirring for 2 minutes, thus obtaining encapsulated pancreatic islets (five-layered membrane formulation 3) covered with a five-layered membrane: an alginic acid membrane, a polyornithine membrane, an alginic acid membrane, polyornithine membrane, and an alginic acid membrane. The resulting encapsulated pancreatic islets were cultured in a CO₂ incubator at 37°C for 25 days.

### 5. Measurement of capsule size

Encapsulated pancreatic islets were placed in a 6-well plate, and the capsules were photographed at 2x magnification using a stereomicroscope (M165FC, Leica). Using the circle analysis tool of Leica Application Suite V3, the diameters of 30 capsules were measured to calculate the average thereof.

### 6. Measurement of capsule strength

20 to 50 encapsulated pancreatic islets were placed in a 35-mm dish, and crushed from above using a viscosity-measuring device; and the force at which the capsules were destroyed was measured. The applied force was divided by the number of capsules, thus obtaining the strength per capsule.

### 7. Measurement of amount of embedding of pancreatic islets

Pancreatic islets were placed in a 6-well plate, and the number of pancreatic islets with a size of 50 µm or more was counted in 50 µm increments. By individually multiplying the number of pancreatic islets by a factor determined for each size, IEQ was calculated. The sum of the IEQs was determined as the total IEQ, and adjusted to 16,000 IEQ. Sodium alginate was then added.

### 8. Evaluation of efficacy using diabetic mice

A small incision was made along the midline of the abdomen of each of the C57BL/6J mice to which diabetes had been induced by STZ. Encapsulated pancreatic islets suspended in a physiological saline solution were transplanted thereto at a dose of 10,000 IEQ/body using a sterile dropper. After transplantation, the mice were kept for 12 weeks, and blood was collected from the tail vein of each mouse to measure the blood glucose level over time.

As shown in Fig. 1, it was confirmed that the administration of the five-layered membrane formulation inhibited an increase in blood glucose level in diabetic mice for a long period of time, as compared to the administration of the three-layered membrane formulation.

### 9. Measurement of fibrosis rate

A small incision was made along the midline of the abdomen of each of the healthy C57BL/6J mice. Encapsulated pancreatic islets were transplanted thereto at a dose of 1,000 capsules/body using a sterile dropper. One week after the transplantation, from the abdominal cavity, the encapsulated pancreatic islets were collected in a 125-mL container containing a Hanks' Balanced Salt Solution (HBSS). The supernatant was removed, followed by washing with HBSS. The encapsulated pancreatic islets that had been suspended were collected in a 35-mm dish, and the number of capsules covered with fibroblasts was counted. The number of fibrosis capsules was divided by the number of total capsules in the dish, and the average of two dishes was expressed as the fibrosis rate.

As shown in Fig. 2, it was confirmed that when the encapsulated pancreatic islet had a diameter more than about 400 µm, the fibrosis was significantly inhibited.

## Claims

1. A formulation comprising a core covered with a five-layered membrane, wherein
the core comprises one or more pancreatic islets, and
the first, third, and fifth membranes from the inner side of the five-layered membrane contain alginic acid, and the second and fourth membranes from the inner side contain polyornithine.

2. The formulation according to claim 1, wherein the formulation has an average diameter of 400 µm or more, measured by microscopy in accordance with by the section *"Measurement of capsule size"* of the specification.

3. The formulation according to claim 1 or 2, wherein the formulation has an average diameter of 400 µm or more to 500 µm or less.

4. The formulation according to any one of claims 1 to 3, wherein the one or more pancreatic islets are pancreatic islets obtained from a one- to three-week-old neonatal pig.

5. The formulation according to any one of claims 1 to 4 for use in the treatment of diabetes.

6. A method for producing the formulation according to any one of claims 1 to 4, comprising the steps of:
(a) preparing a sodium alginate solution A containing one or more pancreatic islets;
(b) adding the sodium alginate solution A to a divalent cation solution dropwise, and collecting gelled particles;
(c) adding the particles collected in step (b) to a poly-L-ornithine solution A, followed by stirring for a predetermined period of time and then collecting particles;
(d) adding the particles collected in step (c) to a sodium alginate solution B, followed by stirring for a predetermined period of time and then collecting particles;
(e) adding the particles collected in step (d) to a poly-L-ornithine solution B, followed by stirring for a predetermined period of time and then collecting particles;
(f) adding the particles collected in step (e) to a sodium alginate solution C, followed by stirring for a predetermined period of time and then collecting particles; and
(g) adding the particles collected in step (f) to a sodium citrate solution, followed by stirring for a predetermined period of time and then collecting particles.

7. The method according to claim 6, wherein the poly-L-ornithine solution A has a poly-L-ornithine concentration of 0.05 w/v% or more to less than 1 w/v%.

8. The method according to claim 6 or 7, wherein in step (d), the particles collected in step (c) are added to the sodium alginate solution B after being added to a poly-L-ornithine solution A'**,** stirred for a predetermined period of time, and then collected.

## Patentansprüche

1. Formulierung, umfassend einen Kern, der mit einer fünfschichtigen Membran bedeckt ist,
wobei
der Kern eine oder mehrere Pankreasinseln umfasst, und
die erste, die dritte und die fünfte Membran von der Innenseite der fünfschichtigen Membran aus Alginsäure enthalten und die zweite und die vierte Membran von der Innenseite aus Polyornithin enthalten.

2. Formulierung gemäß Anspruch 1, wobei die Formulierung einen durchschnittlichen Durchmesser von 400 µm oder mehr, gemessen durch Mikroskopie gemäß dem Abschnitt *"Measurement of capsule size"* der Beschreibung, aufweist.

3. Formulierung gemäß Anspruch 1 oder 2, wobei die Formulierung einen durchschnittlichen Durchmesser von 400 µm oder mehr bis 500 µm oder weniger aufweist.

4. Formulierung gemäß einem der Ansprüche 1 bis 3, wobei die eine oder mehreren Pankreasinseln Pankreasinseln, erhalten von einem ein bis drei Wochen alten neugeborenen Schwein, sind.

5. Formulierung gemäß einem der Ansprüche 1 bis 4 zur Verwendung bei der Behandlung von Diabetes.

6. Verfahren zur Herstellung der Formulierung gemäß einem der Ansprüche 1 bis 4, umfassend die Schritte:
(a) das Herstellen einer Natriumalginat-Lösung A, enthaltend eine oder mehrere Pankreasinseln;
(b) das tropfenweise Hinzufügen der Natriumalginat-Lösung A zu einer Lösung zweiwertiger Kationen und das Sammeln der gelierten Partikel;
(c) das Hinzufügen der in Schritt (b) gesammelten Partikel zu einer Poly-L-Ornithin-Lösung A, gefolgt von Rühren für eine vorbestimmte Zeitdauer und anschließendem Sammeln der Partikel;
(d) das Hinzufügen der in Schritt (c) gesammelten Partikel zu einer Natriumalginat-Lösung B, gefolgt von Rühren für eine vorbestimmte Zeitdauer und anschließendem Sammeln der Partikel;
(e) das Hinzufügen der in Schritt (d) gesammelten Partikel zu einer Poly-L-Ornithin-Lösung B, gefolgt von Rühren über einen vorbestimmten Zeitraum und anschließendem Sammeln der Partikel;
(f) das Hinzufügen der in Schritt (e) gesammelten Partikel zu einer Natriumalginat-Lösung C, gefolgt von Rühren über einen vorbestimmten Zeitraum und anschließendem Sammeln der Partikel; und
(g) das Hinzufügen der in Schritt (f) gesammelten Partikel zu einer Natriumcitrat-Lösung, gefolgt von Rühren über einen vorbestimmten Zeitraum und anschließendem Sammeln der Partikel.

7. Verfahren gemäß Anspruch 6, wobei die Poly-L-Ornithin-Lösung A eine Poly-L-Ornithin-Konzentration von 0,05% (w/v) oder mehr bis weniger als 1% (w/v) aufweist.

8. Verfahren gemäß Anspruch 6 oder 7, wobei in Schritt (d) die in Schritt (c) gesammelten Partikel zu der Natriumalginat-Lösung B hinzugefügt werden, nachdem sie zu einer Poly-L-Ornithin-Lösung A' gegeben, für eine vorbestimmte Zeit gerührt und anschließend gesammelt wurden.

## Revendications

1. Formulation comprenant un noyau recouvert d'une membrane à cinq couches, dans laquelle
le noyau comprend un ou plusieurs îlots pancréatiques, et
les première, troisième et cinquième membranes à partir du côté intérieur de la membrane à cinq couches contiennent de l'acide alginique, et les deuxième et quatrième membranes à partir du côté intérieur contiennent de la polyornithine.

2. Formulation selon la revendication 1, dans laquelle la formulation présente un diamètre moyen supérieur ou égal à 400 µm, mesuré par microscopie conformément à la section « *Mesure des tailles de capsules »* de la description.

3. Formulation selon la revendication 1 ou 2, dans laquelle la formulation présente un diamètre moyen supérieur ou égal à 400 µm et inférieur ou égal à 500 µm.

4. Formulation selon l'une quelconque des revendications 1 à 3, dans laquelle l'un ou plusieurs îlots pancréatiques sont des îlots pancréatiques obtenus à partir d'un porcelet nouveau-né âgé d'une à trois semaines.

5. Formulation selon l'une quelconque des revendications 1 à 4, destinée à une utilisation dans le traitement du diabète.

6. Procédé destiné à produire la formulation selon l'une quelconque des revendications 1 à 4, comprenant les étapes suivantes :
(a)préparer une solution d'alginate de sodium A contenant un ou plusieurs îlots pancréatiques ;
(b) ajouter goutte à goutte la solution d'alginate de sodium A à une solution de cation divalent et recueillir les particules gélifiées ;
(c) ajouter les particules recueillies à l'étape (b) à une solution de poly-L-ornithine A, puis agiter pendant une période de temps prédéterminée, puis recueillir les particules ;
(d) ajouter les particules recueillies à l'étape (c) à une solution d'alginate de sodium B, puis agiter pendant une période de temps prédéterminée, puis recueillir les particules ;
(e) ajouter les particules recueillies à l'étape (d) à une solution de poly-L-ornithine B, puis agiter pendant une période de temps prédéterminée, puis recueillir les particules ;
(f) ajouter les particules recueillies à l'étape (e) à une solution d'alginate de sodium C, puis agiter pendant une période de temps prédéterminée, puis recueillir les particules ; et
(g) ajouter les particules recueillies à l'étape (f) à une solution de citrate de sodium, puis agiter pendant une période de temps prédéterminée, puis recueillir les particules.

7. Procédé selon la revendication 6, dans lequel la solution de poly-L-ornithine A présente une concentration en poly-L-ornithine supérieure ou égale à 0,05 % p/v et inférieure à 1 % p/v.

8. Procédé selon la revendication 6 ou 7, dans lequel, à l'étape (d), les particules recueillies à l'étape (c) sont ajoutées à la solution d'alginate de sodium B après avoir été ajoutées à une solution de poly-L-ornithine A', agitées pendant une période de temps prédéterminée, puis recueillies.
